(19) Europäisches Patentamt
European Patent Office
Office européen des brevets

(11) EP 3 440 053 B1

(12) **FASCICULE DE BREVET EUROPEEN**

(45) Date de publication et mention de la délivrance du brevet:
**27.09.2023 Bulletin 2023/39**

(21) Numéro de dépôt: **17715188.3**

(22) Date de dépôt: **07.04.2017**

(51) Classification Internationale des Brevets (IPC):
***C07C 263/10*** $^{(2006.01)}$ ***C07C 263/20*** $^{(2006.01)}$
***C07C 265/14*** $^{(2006.01)}$

(52) Classification Coopérative des Brevets (CPC):
(C-Sets disponibles)
**C07C 263/10; C07C 263/20** (Cont.)

(86) Numéro de dépôt international:
**PCT/EP2017/058341**

(87) Numéro de publication internationale:
**WO 2017/174765 (12.10.2017 Gazette 2017/41)**

(54) **PROCÉDÉ DE PRÉPARATION DES XYLYLÈNE DIISOCYANATES XDI**

VERFAHREN ZUR HERSTELLUNG VON XYLYLEN-DIISOCYANAT (XDI)

METHOD FOR PRODUCING XYLYLENE DIISOCYANATE (XDI)

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **07.04.2016 FR 1653051**

(43) Date de publication de la demande:
**13.02.2019 Bulletin 2019/07**

(73) Titulaire: **Vencorex France**
**69800 Saint-Priest (FR)**

(72) Inventeurs:
- **RATABOUL-LEDUC, Anne-Marie**
  **69008 LYON (FR)**
- **BERNARD, Jean-Marie**
  **69440 SAINT LAURENT D'AGNY (FR)**
- **TOURNAYRE, Julien**
  **69540 Irigny (FR)**

(74) Mandataire: **Dennemeyer & Associates S.A.**
**Landaubogen 1-3**
**81373 München (DE)**

(56) Documents cités:
**WO-A1-2009/077795 FR-A- 1 555 515**
**US-A- 3 658 656**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

(52) Classification Coopérative des Brevets (CPC):
(Cont.)

C-Sets
**C07C 263/10, C07C 265/14;**
**C07C 263/20, C07C 265/14**

## Description

DOMAINE TECHNIQUE

L'invention se rapporte à un procédé de préparation des xylylène diisocyanates de formule $R(NCO)_2$ (R étant le noyau diméthylbenzénique), en abrégé XDI, en particulier du méta-xylylène diisocyanate en abrégé mXDI, par phosgénation de la xylylène diamine (ou XDA) correspondante. L'invention se rapporte également au produit XDI ainsi obtenu. Les XDI peuvent également être préparés sans phosgénation, par exemple à partir de dicarbamate de xylylène, de diformamide de xylylène, ou de dihalogénure de xylylène. Ces derniers types de préparation n'entrent pas dans le domaine de la présente invention.

Le XDI est un diisocyanate araliphatique dont les propriétés sont intermédiaires entre les diisocyanates (cyclo)aliphatiques - tels que le diisocyanate d'hexaméthylène (HDI) ou d'isophorone (IPDI) - et les diisocyanates aromatiques - tels que le diisocyanate de toluène (TDI) ou de diphényl-méthane (MDI). Dans le présent texte, ce qui concerne le mXDI se rapporte également, sauf indication contraire, à l'ortho- et au para-xylylène diisocyanate et donc se rapporte de manière générale à un XDI.

La polycondensation des diisocyanates avec des polyols, des polyamines ou des polythiols intervient dans la réalisation de produits polyuréthane, polyurée, polythiouréthane plus spécifiquement pour les domaines de la peinture, des adhésifs, des matériaux transparents ou de verre optique. Dans ce dernier cas, les verres optiques fabriqués à partir du mXDI ont un indice de réfraction particulièrement élevé ce qui permet, à performance égale, de réaliser des lentilles de plus faible épaisseur (ou de produire des lentilles de meilleures performances à épaisseur égale).

Pour une application donnée, le choix d'un isocyanate s'effectue en fonction des propriétés recherchées et de la réactivité souhaitée. Alors que les diisocyanates (cyclo)aliphatiques (HDI, IPDI) présentent une forte résistance au jaunissement associée à une faible réactivité, les diisocyanates aromatiques (TDI, MDI) sont sensiblement plus réactifs mais jaunissent rapidement. L'intérêt des XDI, et en particulier du mXDI, est, d'une part de présenter une résistance au jaunissement supérieure aux isocyanates aromatiques et, d'autre part, d'avoir des fonctions isocyanates ayant une réactivité intermédiaire entre celles des isocyanates aliphatiques et aromatiques. Ces propriétés assurent une réactivité et une durée de vie compatibles avec certaines applications ciblées, par exemple comme film d'emballage alimentaire. En effet, pour ces applications, une durée de vie limitée, par exemple 6 mois, est tout à fait compatible avec la durée de vie de leur utilisation.

Classiquement, le XDI, comme les autres diisocyanates, est préparé par réaction du phosgène $COCl_2$ sur une diamine, ici une xylylène-diamine $R(NH_2)_2$, par exemple la m-xylylène-diamine pour préparer le mXDI. Cette réaction de phosgénation conduit à la transformation de la diamine et se déroule en deux étapes : une étape de formation de produits intermédiaires, en particulier des chlorures de carbamyle, suivie d'une étape de transformation de ces produits intermédiaires en isocyanates - à une température de réaction classiquement comprise dans l'intervalle 130-170°C - et à pression comprise entre la pression atmosphérique et une pression d'environ 4 bars.

Ainsi, de manière simplifiée, une première étape peut produire principalement des chlorures de carbamyle et de l'acide chlorhydrique par réaction des fonctions amines avec le phosgène selon la réaction-type:

$$R(NH_2)_2 + 2COCl_2 \dashrightarrow R(NHCOCl)_2 + 2HCl \qquad (I)$$

et la deuxième étape fournit du XDI par décomposition des chlorures de carbamyle conformément à la réaction de déshydrochloration suivante:

$$R(NHCOCl)_2 \dashrightarrow R(NCO)_2 + 2\ HCl \qquad (II)$$

Après purification, le rendement en XDI est compris entre 91 et 95%.

ÉTAT DE LA TECHNIQUE

Il est possible de réaliser la phosgénation par différentes voies, en fonction des conditions de température et de pression, que ce soit en phase totalement liquide, partiellement liquide et partiellement gazeuse, ou totalement gazeuse. Suivant ces conditions, des produits intermédiaires sont constitués de mélanges de différents chlorhydrates d'amine et/ou de différents chlorures de carbamyle dans des proportions variées.

En particulier, une meilleure réactivité peut être obtenue pour la préparation d'isocyanates par une étape préalable de chlorhydratation de la diamine mXDA par réaction avec l'acide chlorhydrique. Le produit intermédiaire obtenu - le chlorhydrate d'amine correspondant $R(NH_2.HCl)_2$ - est alors soumis, dans la deuxième étape de phosgénation, à une température de réaction élevée, par exemple 190°C, comme décrit par exemple dans le document de brevet US 5 523 467.

Selon un autre exemple, le procédé décrit dans CN 102070491 prévoit une étape de formation d'un sel par chlorhydratation d'une solution de XDA dans un mélange de solvant inerte et d'acide chlorhydrique. Cette étape est suivie d'une étape de concentration par centrifugation du chlorhydrate formé, puis d'étapes de phosgénation à haute et basse pression. Dans ce procédé, la réaction de chlorhydratation de l'amine est maintenue à faible concentration en solution et le chlorhydrate est concentré pour préparer le XDI. Ce procédé assure un taux de conversion élevé à partir d'une solution de chlorhydrates d'amine.

D'autres améliorations ont été obtenues par des traitements particuliers. L'utilisation d'un reflux de dichlorobenzène permet de baisser la température de réaction à 120-125°C avec un bon rendement (par exemple 91,4%) et un bon degré de pureté (par exemple 99,3 %). De plus, conduire la réaction sous une atmosphère d'azote améliore le rendement (92,9%), comme rapporté dans le brevet US 5 523 467 déjà cité. Par ailleurs, une augmentation de pression, jusqu'à 2 bars, lors de la réaction de l'acide chlorhydrique sur la diamine mXDA établit un rendement encore meilleur (98,8%) mais un degré de pureté sensiblement inférieur (98,4%). Cependant, ce type de procédé est lent et nécessite une étape de chlorhydratation.

L'étape de phosgénation est généralement suivie d'une étape de purification qui permet d'éliminer le solvant de réaction ainsi que les impuretés pour obtenir le composé isocyanate recherché avec une pureté augmentée. Certaines des espèces à éliminer contiennent en particulier des chlores hydrolysables. Le terme « chlore hydrolysable » fait ici référence aux atomes de chlore labiles présents dans les composés isocyanates, comme défini par exemple dans GB1350374.

Il est également connu que, pour certaines applications finales, notamment dans le domaine des mousses de polyuréthane, la présence de chlore hydrolysable dans l'isocyanate altère les propriétés du produit final.

Pour tenter de réduire la quantité de chlore hydrolysable des isocyanates organiques bruts, US 3219678 propose une purification à haute température - au moins égales à 190°C dans les exemples - entre deux distillations visant à éliminer respectivement le solvant et des impuretés résiduelles. Cependant cette solution prévoit des températures élevées pour les isocyanates aromatiques alors que ces hautes températures risquent de décomposer les isocyanates araliphatiques, en particulier les XDI.

Il est également possible, pour éliminer le chlore hydrolysable, d'ajouter au préalable des additifs d'élimination ciblés: des métaux et oxydes métalliques, des imidazoles, des acides sulfoniques et leurs esters, le diéthylsulfate, l'acide sulfurique, les trialkylphosphates, des composés époxy. D'autres additifs - comme les composés ayant au moins un groupe NH (urées, biuret, caprolactame, sels d'ammonium, carbodiimides, sels d'amine primaire ou secondaire, etc.) ou des alcools tertiaires - ont été proposés.

Cependant, ces additifs génèrent une décroissance sensible de la quantité de groupes isocyanates, un accroissement de la viscosité, diminuent sensiblement le rendement et nécessitent en général une séparation supplémentaire de l'isocyanate et de l'additif.

De plus, les isocyanates XDI se singularisent des isocyanates aromatiques, en particulier du TDI, par la formation de composés chloroalkyl monoisocyanates, en particulier le chlorométhyl-benzyl-isocynate CIRNCO (ci-après CIBi), et même de dichloroxylène. Le CIBi et ses dérivés proviennent de la substitution d'une fonction isocyanate par un atome de chlore. Ces sous-produits sont particulièrement difficiles à éliminer.

Le CIBi et ses dérivés sont en général présents dans des proportions de 3 à 10% voire 20%, comme indiqué dans le brevet EP 0 384 463. Le CIBi accélère la gélification du pré-polymère et affecte les propriétés de la résine de polyuréthane obtenue. C'est pourquoi les conditions d'empêchement de formation du CIBi et de ses dérivés ont été activement recherchées.

Les recherches ont ainsi porté sur les conditions de phosgénation pour réduire les quantités de sous-produits chlorés. Dans US 3 470 227, la phosgénation a été conduite entre 2 et 5 bars avec une vanne de réglage et un dégazage de l'acide chlorhydrique pour maintenir la pression à une valeur constante.

Toutefois, si la température de réaction de phosgénation dépasse 180°C, afin de diminuer le temps de réaction, la concentration en produits secondaires augmente significativement. L'utilisation d'un solvant de type ester, tel que l'acétate d'héxyle ou l'acétate d'amyle, a alors été préconisée - par exemple dans EP 0 384 463 déjà cité - pour limiter leur formation.

Les recherches ont aussi porté sur les conditions de purification, notamment pour diminuer la teneur en CIBi dans le XDI. Ainsi, afin d'améliorer la séparation du CIBi du XDI, un gaz inerte ou un solvant à contre-courant peut également être mis en oeuvre lors d'une distillation, comme illustré dans FR 1 555 517, GB 1 119 459 ou FR 1 555 515. La température de distillation s'en trouve également diminuée.

De manière générale, il apparaît que, pour la purification des XDI par distillation, l'étape finale d'élimination des espèces légères est délicate car l'impureté principale à éliminer, le CIBi, a une tension de vapeur proche de celle des XDI.

EXPOSÉ DE L'INVENTION

L'invention vise à mettre au point un procédé de préparation optimisé des XDI par phosgénation des amines XDA par voie non chlorhydrate (c'est-à-dire que le procédé de l'invention ne comprend pas d'étapes préalables de préparation de chlorhydrates et ne comprend pas la mise en oeuvre d'espèces chlorhydrates) en proposant de réduire au maximum, voire d'éliminer la présence d'espèces chimiques intermédiaires, tout en maîtrisant la conduite de la réaction, en particulier en diminuant le risque d'emballement thermique, et en permettant d'augmenter sensiblement le rendement. L'invention propose encore de faciliter la séparation des composés issus de la phosgénation, en évitant de catalyser des réactions secondaires conduisant à des composés lourds, tout en diminuant la quantité de déchets à éliminer, en particulier de produits lourds.

Pour ce faire, l'invention propose de favoriser - avant la séparation tendant à éliminer le solvant - la transformation d'espèces chimiques intermédiaires.formées au cours du processus de phosgénation en molécules possédant des fonctions isocyanates avec libération d'HCl.

On entend par « espèces chimiques intermédiaires » les espèces contenant au moins une fonction -NH-CO-Cl, appelée fonction chlorure de carbamyle, ou au moins une fonction -N(-CO-Cl)-CO-NH-, appelée fonction chlorure d'allophanoyle et/ou des fonctions dérivées par combinaison ou condensation de ces espèces entre elles et/ou leur combinaison et pouvant libérer au moins une molécule d'HCl. Les fonctions chlorures de carbamyle et chlorures d'allophanoyles sont en équilibre avec les fonctions isocyanates suivant. les réactions suivantes :

(1) isocyanate + HCl ↔chlorure de carbamyle

(2) isocyanate + chlorure de carbamyle ↔chlorure d'allophanoyle En appelant K1 la constante d'équilibre de la réaction (1) pour le mono et le di-chlorure de carbamyle du mXDI, du TDI et du HDI pris indépendamment et K2 la constante d'équilibre de la réaction (2) pour le chlorure d'allophanoyle du mXDI, du TDI et du HDI pris indépendamment, si l'on compare ces différentes constantes pour différents isocyanates (mXDI, TDI et HDI), il en ressort :

$$K1(mXDI)/K1(TDI) \approx 7$$

$$K1(mXDI)/K1(HDI) \approx 1$$

$$K2(mXDI)/K2(TDI) \approx 8$$

$$K2(mXDI)/K2(HDI) \approx 4$$

Cela montre que, contrairement aux autres isocyanates, le chlorure d'allophanoyle du mXDI est stable. Il y a donc un intérêt à réduire au maximum voire à l'éliminer le plus tôt possible pour ne pas avoir d'impact négatif sur la quantité finale de mXDI. Il y a donc un intérêt à éliminer ou à tout le moins réduire la formation de ces espèces chimiques intermédiaires.

Selon une caractéristique avantageuse, les espèces chimiques intermédiaires transformées durant le procédé selon l'invention et comportant essentiellement des fonctions de chlorure de carbamyle -NHCOCl de XDI, de chlorure d'allophanoyle -N(-CO-Cl)-CO-NH- de XDI et/ou des fonctions dérivées par combinaison ou condensation de ces espèces entre elles, présentent de préférence des fonctions ayant l'une et/ou l'autre des formules développées respectivement de la forme:

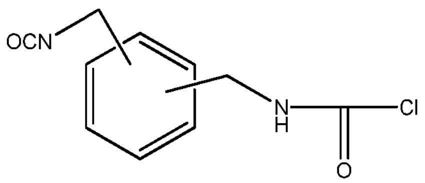

monochlorure de carbamyle de XDI

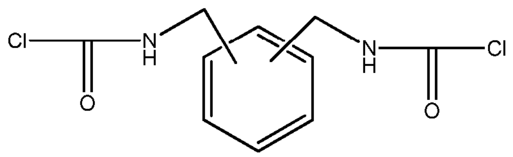

dichlorure de carbamyle de XDI

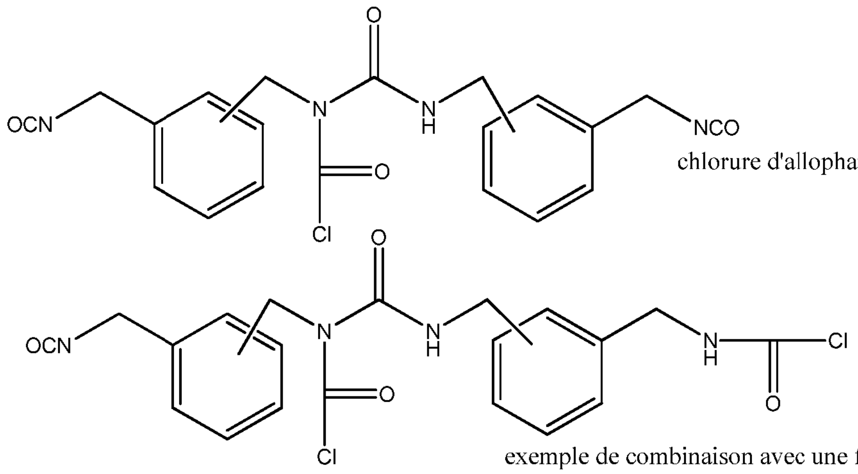

chlorure d'allophanoyle de XDI

exemple de combinaison avec une fonction chlorure d'allophanoyle et chlorure de carbamyle

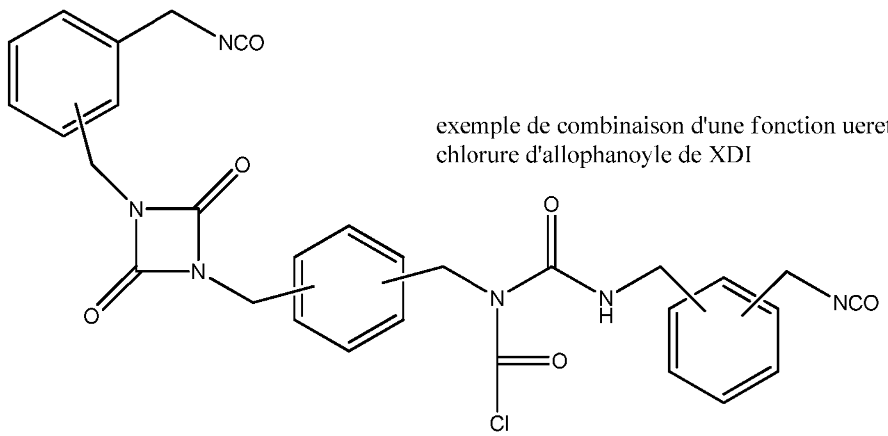

exemple de combinaison d'une fonction ueretinedione et chlorure d'allophanoyle de XDI

Ces espèces chimiques intermédiaires peuvent également contenir d'autres fonctions, telles que des fonctions urétidinedione (encore appelées dimère), isocyanurate, carbodiimide, iminouretidinedione, iminotriazinedione et/ou oxadiazinetrione, ainsi que leur combinaison avec les chlorures de carbamyle et d'allophanoyle.

Les inventeurs ont constaté, de manière surprenante, que plus la transformation de ces espèces chimiques intermédiaires intervient tôt dans le procédé réactionnel, plus le rendement en XDI est élevé. Par ailleurs, les inventeurs ont également remarqué, de manière surprenante, que la présence d'espèces chimiques intermédiaires persistent en fin de réaction de phosgénation des XDA, après l'élimination du phosgène alors que, classiquement, pour les diisocyanates aromatiques ou aliphatiques, comme l'HDI, le TDI ou l'IPDI, de telles espèces chimiques intermédiaires peuvent se détecter en cours de préparation mais ne sont quasiment plus présentes à l'issue de la phosgénation.

De plus, les inventeurs ont remarqué, de manière surprenante, que la présence d'espèces chimiques intermédiaires abaisse significativement la température seuil d'emballement thermique dans les étapes de purification qui suivent l'élimination du solvant. Par ailleurs, les inventeurs ont constaté que plus la quantité d'espèces chimiques intermédiaires est élevée, plus la température seuil de décomposition thermique est abaissée. Cela entraîne un risque pour la sécurité lors de la purification du XDI.

L'invention propose alors d'éliminer du milieu réactionnel au plus tôt l'acide chlorhydrique dans des conditions spécifiques permettant un déplacement maîtrisé de la réaction (1) vers la production d'isocyanate, afin de produire en plus grande quantité le composé isocyanate recherché et de limiter la formation d'espèces chimiques intermédiaires.

L'invention a plus précisément pour objet un procédé de préparation des xylylène diisocyanates XDI, en particulier du méta-xylylènediisocyanate mXDI, comprenant les étapes de :

a. phosgénation de la xylylène-diamine XDA, en particulier de la m-xylylène diamine mXDA dans le cas du mXDI ;
b. élimination de l'acide chlorhydrique du milieu réactionnel obtenu à l'étape a) à une température comprise entre 120 et 190°C et à une pression comprise entre 1 mbar et c. purification du XDI obtenu comprenant par exemple une étape d'élimination du $COCl_2$, une étape d'élimination du solvant de phosgénation, une étape d'élimination des composés lourds formés lors de la phosgénation et une étape d'élimination des espèces légères formées lors de la phosgénation.

L'étape b) est maintenue jusqu'à ce que le dosage du milieu réactionnel résultant indique une teneur en chlorure d'allophanoyle de XDI, de préférence de mXDI, inférieure à 3%, de préférence inférieure à 2%, avantageusement inférieure à 1%, en masse dans le milieu réactionnel, en excluant la quantité de solvant, de phosgène et d'HCl.

L'étape b) est une étape d'élimination de l'acide chlorhydrique formé lors du procédé, ce qui permet avantageusement de déplacer l'équilibre des réactions (1) et (2) décrites ci-dessus vers la formation de XDI. De préférence, l'élimination de l'acide chlorhydrique est opérée dans des conditions de température ajustées de sorte à éviter un emballement thermique.

L'étape b), qui favorise la transformation des espèces chimiques intermédiaires, permet de diminuer sensiblement la quantité des sous-produits chlorés, favorise la baisse des coûts de maintenance grâce à un encrassement moindre de l'installation, ce qui permet également un bénéfice environnemental par la diminution des déchets à brûler.

La transformation des espèces chimiques intermédiaires possédant des fonctions chlorure d'allophanoyle est un marqueur de la transformation des espèces chimiques intermédiaires possédant des fonctions chlorure de carbamyle. C'est pourquoi la quantité de chlorure d'allophanoyle du XDI est suivie durant la mise en oeuvre du procédé de l'invention.

Les composés chlorure de carbamyle et chlorure d'allophanoyle sont des composés qui par hydrolyse libèrent des chlorures. Les chlorures libérés, qui permettent de déterminer la quantité de chlorure de carbamyle et chlorure d'allophanoyle, sont obtenus en faisant réagir, selon la méthode de la norme ISO 15028 :2014, le milieu réactionnel avec une solution hydroalcoolique chaude. La teneur en chlore libéré est ensuite mesurée par titrage par toute méthode connue de l'homme du métier, notamment par la mise en oeuvre d'une solution de nitrate d'argent.

Le procédé de l'invention comprend après l'étape b), les étapes classiques de purification du XDI obtenu. Notamment, le procédé de l'invention comprend, après l'étape b) une étape d'élimination du $COCl_2$, une étape d'élimination du solvant de phosgénation, une étape d'élimination des composés lourds formés lors de la phosgénation (composés ayant un point d'ébullition strictement supérieur au XDI comme par exemple le dimère ou trimère de XDI) et une étape d'élimination des espèces légères formées lors de la phosgénation (composés ayant un point d'ébullition strictement inférieur au XDI comme par exemple le CIBi). Ces étapes sont classiquement connues de l'homme du métier et sont mises en oeuvre dans les conditions et avec les dispositifs classiquement connus de l'homme du métier. Avantageusement, pour éviter toute possibilité d'emballement thermique, l'étape b) est opérée dans un solvant et à une température supérieure à 120°C et inférieure à 190°C.

Le solvant de l'étape b) est de préférence choisi parmi les solvants non réactifs avec les fonctions isocyanates et notamment compatibles avec les conditions de mise en oeuvre. De préférence, le solvant est choisi parmi les alcanes, les chloroalcanes, les esters, les éthers, les aromatiques et les aromatiques halogénés. Avantageusement, le solvant de l'étape d'élimination de l'acide chlorhydrique peut être le solvant utilisé pour la réaction de phosgénation.

Comme mentionné plus haut, l'étape b) permet de manière avantageuse de transformer les espèces chimiques intermédiaires de manière précoce. Réalisée après la réaction de phosgénation et avant les étapes ultérieures de séparation du solvant, des composés lourds et des espèces légères, cette étape permet de travailler sans risque d'emballement thermique et de dégradation des produits issus de la réaction de phosgénation. Cette transformation des espèces chimiques intermédiaires avant séparation permet de plus d'augmenter le rendement en XDI global.

En conséquence, il apparaît que les étapes de séparation du solvant et des composés lourds, puis les étapes ultérieures de séparation des espèces légères, en particulier du mélange CIBi/XDI, peuvent être effectuées dans des domaines de température et de pression plus larges. En effet lorsque les espèces chimiques intermédiaires ont été préalablement transformées, la stabilité thermique des différents milieux est améliorée permettant ainsi une conduite favorisée des différents équipements et une meilleure productivité.

De manière avantageuse, avant l'étape b), le milieu réactionnel issu de l'étape a) est dilué dans le solvant à raison d'une dilution supérieure à 30% en poids, de préférence supérieure à 50% en poids. Cette dilution permet de façon avantageuse de réduire la génération de réactions secondaires.

Dans un premier mode de réalisation, l'étape b) consiste à mettre en contact, à co-courant, à courant croisé ou à contre-courant, le milieu réactionnel issu de l'étape a), optionnellement dilué comme précisé ci-dessus, et d'au moins un composé inerte choisi de préférence parmi un composé de diazote, d'argon, de gaz carbonique et d'alcane léger de C1 à C4, dans un état vapeur ou gazeux dans les conditions opératoires de température comprise entre 120°C et 190°C, de préférence entre 150°C et 190°C, et de pression absolue comprise entre 1 et 5 bars, de préférence entre 1 et 3 bar. Cette étape est mise en oeuvre dans un contacteur gaz-liquide et de préférence dans une colonne de stripping.

Dans un autre mode de réalisation, l'étape b) est réalisée par évaporation, en batch ou en continu, de l'acide

chlorhydrique contenu dans le milieu réactionnel issu de l'étape a), éventuellement dilué comme précisé ci-dessus, à une température comprise entre 25°C et 140°C, de préférence entre 80 et 140°C, et une pression comprise entre 10 et 1100 mbars absolus.

Dans un autre mode de réalisation, l'étape b) est réalisée par piégeage chimique de l'acide chlorhydrique contenu dans le milieu réactionnel issu de l'étape a), éventuellement dilué comme précisé ci-dessus, par des amines tertiaires pouvant être immobilisées sur de la silice ou une base sans hydrogène mobile.

Dans un autre mode de réalisation, l'étape b) se fait par adsorption de l'acide chlorhydrique, notamment par mise en contact du milieu réactionnel issu l'étape a), éventuellement dilué comme précisé ci-dessus, avec un substrat solide constitué par des zéolithes ou des résines échangeuses d'ions, en particulier de type amine quaternaire, à une température comprise entre 20 °C et 140 °C, l'acide chlorhydrique restant fixé sur la surface du solide et le liquide ressortant purifié sans acide chlorhydrique.

Dans un autre mode de réalisation, l'étape b) est effectuée par séparation du milieu réactionnel issu de l'étape a), éventuellement dilué comme précisé ci-dessus, par une technique membranaire, en particulier par pervaporation selon une vaporisation spécifique de l'acide chlorhydrique au travers d'une membrane organique ou céramique, avec une température comprise entre 30 et 160°C, et une pression partielle d'acide chlorhydrique dans le perméat comprise entre 0.1 et 100 mbar absolu, et/ou une migration par perméation spécifique de l'acide chlorhydrique et rétention des autres espèces grâce à une membrane organique ou céramique, à une température comprise entre 20 et 90°C.

Dans un autre mode de réalisation, l'étape b) correspond à une distillation du milieu réactionnel issu de l'étape a) à une température comprise entre 120 et 190°C, de préférence entre 140 et 190°C, et à une pression comprise entre 1 et 20 bars, de préférence entre 12 et 17 bars. De préférence, la distillation est réalisée dans une colonne associée à un bouilleur et un condenseur avec, en tête, le retrait de l'acide chlorhydrique, puis la récupération en pied du milieu réactionnel purifié. De préférence, la distillation a lieu en batch ou en continu. De préférence, la température du milieu dans le bouilleur lors de la distillation est comprise entre 120 et 190°C, de préférence entre 140 et 190°C, et la pression en tête de la colonne à distiller comprise entre 1 et 20 bars absolus, de préférence entre 12 et 17 bars absolus.

Avantageusement, l'étape b) et chaque étape de séparation sont effectuées en présence d'un inhibiteur de polymérisation.

La séparation finale entre le ClBi et le XDI se fait à une température plus élevée, entre 150 et 190°C, avec une pression moindre, entre 4-5 mbar et 15 à 20 mbars, En conséquence la conduite de la distillation en est facilitée, sans risque pour la sécurité de l'installation, et les coûts en sont réduits. De façon particulièrement avantageuse, il est noté que l'étape d'élimination de l'acide chlorhydrique permet une meilleure séparation du ClBi du XDI.

L'invention se rapporte également à une composition de XDI, en particulier de mXDI, notamment préparée par la mise en oeuvre du procédé défini ci-dessus caractérisé par une teneur en chlorure d'allophanoyle de XDI inférieure à 3 %, de préférence inférieure à 2 %, et avantageusement inférieure à 1 % en poids en excluant la quantité de solvant, de phosgène et d'HCl et dans laquelle des résidus de solvant, de composés lourds et d'espèces légères sont encore présents en combinaison ou en mélange après séparation selon un procédé de séparation défini ci-dessus avec des teneurs inférieures à 5%, de préférence inférieures à 3%, et avantageusement inférieures à 1,5%.

Selon un mode de mise en oeuvre particulier, le XDI est stabilisé avec des phénols encombrés en position 2,6 tel que les ionols et ou un inhibiteur de polymérisation de nylon-1, en particulier pendant la période de stockage.

## PRÉSENTATION DES FIGURES

D'autres données, caractéristiques et avantages de la présente invention apparaîtront à la lecture de la description détaillée suivante, d'exemples de réalisation non limités, en référence aux figures annexées, qui représentent respectivement :

- la figure 1, une vue en coupe schématique d'une colonne de stripping pour réaliser l'étape b) par mise en contact à contre-courant avec un gaz inerte;
- la figure 2, une vue en coupe schématique d'un dispositif de séparation membranaire pour la réalisation de l'étape b) par pervaporation ;
- la figure 3, une vue en coupe schématique d'un dispositif à substrat solide pour la réalisation de l'étape b) par fixation/absorption/rétention sélective de l'acide chlorhydrique ;
- la figure 4, une vue en coupe schématique d'un ensemble de distillation comportant une colonne associée à un bouilleur et à un condenseur.

## DESCRIPTION DÉTAILLÉE

Un premier exemple de traitement du mélange réactionnel issu de la phosgénation est illustré par la vue en coupe schématique de la colonne 100 de la figure 1. Le traitement consiste ici à mettre en contact à contre-courant le

mélange liquide issu de la réaction de phosgénation et un composé inerte d'azote à 120°C sous une pression absolue de 2 bars. Cette mise en contact provoque le stripping des molécules d'acide chlorhydrique par transfert dans la phase gazeuse.

Pour ce faire, le mélange réactionnel est d'abord dilué par ajout de 60% en poids de solvant avant d'être introduit en tête de colonne 101 par l'entrée 103. Le composé inerte d'azote est injecté en pied de colonne 104 par l'entrée 106 sous forme gazeuse. L'acide chlorhydrique ressort en tête de colonne 101 avec l'azote par la sortie 105. En pied de colonne, le mélange - purifié d'acide chlorhydrique - est récupéré en sortie 108. Il possède une teneur en solvant de l'ordre de 60% en poids et une teneur en chlorure d'allophanoyle de XDI de l'ordre de 1 % en poids en excluant la quantité de solvant, de phosgène et d'HCl. Cette dernière teneur est suivie par le dosage décrit plus loin.

Un autre exemple de réalisation de l'étape b) est illustré par le dispositif membranaire de séparation 200 de la vue en coupe schématique de la figure 2. Le mélange réactionnel issu de la phosgénation est fortement dilué par ajout de 60% de solvant et pénètre dans ledit dispositif 200 par une entrée 201. Ledit dispositif 200 contient une membrane de séparation 210 qui est de type organique ou, alternativement, de type céramique.

Au contact de cette membrane 210, l'acide chlorhydrique est séparé dudit mélange par pervaporation selon une vaporisation spécifique de l'acide chlorhydrique au travers de la membrane 210, avec une température comprise entre 30 et 160°C, et une pression partielle d'acide chlorhydrique dans le perméat comprise entre 0,1 et 100 mbar absolu.

L'acide chlorhydrique migre alors à travers la membrane 210, mu par la différence de volatilité pour une pervaporation et mu par un gradient de pression pour une perméation. L'acide chlorhydrique est ensuite récupéré en sortie 202, alors que le retentât du mélange initial - purifié des espèces chimiques intermédiaires transformés en XDI - est récupéré par la sortie 204. Le retentât contient 50% en poids de solvant, et la teneur en chlorure d'allophanoyle est inférieur à 2 %, de préférence 1 % selon le dosage de contrôle décrit plus loin. Ce pourcentage correspond au pourcentage massique de chlorure d'allophanoyle du XDI dans le milieu réactionnel en excluant la quantité de solvant, de phosgène et d'HCl.

En particulier,, la séparation peut être réalisée par perméation spécifique de l'acide chlorhydrique et rétention des autres espèces grâce à une membrane organique ou céramique de rétention, la température étant comprise entre 20 et 90°C,avantageusement à 60°C.

Un autre exemple de réalisation de l'étape b) est maintenant présenté, en référence à la vue en coupe schématique du dispositif à substrat solide 300 de la figure 3. Le mélange initial issu de la phosgénation, dilué par un ajout de solvant portant à 70% en poids sa teneur entre dans le dispositif 300 par une entrée 301. Le dispositif 300 contient une zéolithe 310 formant le substrat solide ayant une bonne affinité avec l'acide chlorhydrique. L'acide chlorhydrique est alors adsorbé par fixation à la surface de la zéolithe. La température est fixée entre 20 et 140°C, à 90°C dans l'exemple illustré, et le substrat solide est remplacé lorsqu'il est saturé en acide chlorhydrique. Ce type de substrat solide peut être régénéré et réutilisé.

Alternativement, le substrat solide peut être remplacé par des résines échangeuses d'ions, en particulier de type amine tertiaire ou quaternaire - de préférence tertiaire - peuvent être avantageusement utilisées.

Le mélange purifié d'acide chlorhydrique est récupéré en sortie 402. Ce mélange possède une teneur en chlorure d'allophanoyle de l'ordre de 2% selon le suivi par dosage mis en oeuvre, tel qu'exposé ci-après. Ce pourcentage correspond au pourcentage massique de chlorure d'allophanoyle du XDI dans le milieu réactionnel à 100 % d'extrait sec.

En référence à la vue en coupe schématique de la figure 4, et dans un autre mode de réalisation de l'étape b), une colonne 1 est associée à un bouilleur 2 et à un condenseur 3 pour distiller le mélange réactionnel purifié d'acide chlorhydrique selon l'une des méthodes précédentes. Différents plateaux P1 à P4 sont symboliquement illustrés à différents niveaux de la colonne 1. Cet ensemble de distillation accueille ici un mélange réactionnel issu de la phosgénation d'une solution de méta-xylylène diamine ou mXDA, par le circuit d'alimentation 4 situé à un niveau moyen de la colonne 1.

La réaction de phosgénation génère la formation d'espèces chimiques intermédiaires, en particulier de chlorures d'allophanoyle et de carbamyle de mXDI. Pour réaliser le traitement de transformation des espèces chimiques intermédiaires en mXDI, le milieu réactionnel est repris avec le solvant utilisé pour la réaction de phosgénation, à savoir le monochlorobenzène dans l'exemple illustré. Avantageusement, le solvant est ajouté en grande quantité, égale à 60% en poids du mélange initial dans l'exemple, afin d'empêcher la génération de composés lourds à partir des espèces chimiques intermédiaires.

Le bouilleur 2 est réglé de sorte à établir une température de bas de colonne 12 sensiblement égale à 150°C dans l'exemple illustré. En tête de retrait de colonne 12, les produits légers sont extraits en sortie 14 : l'acide chlorhydrique résiduel ainsi que le phosgène - au moins en partie - et une partie du solvant formant, après passage dans le condenseur 3, le distillat 16 et le reflux 18. En pied de colonne 12, les produits lourds sont recyclés en circuit de recyclage 15 et le résidu - purifié d'acide chlorhydrique - est récupéré en sortie 17. Il contient environ 60 % en poids de solvant et possède une teneur en chlorure d'allophanoyle de XDI inférieure à 1 %. Ce pourcentage correspond au pourcentage massique de chlorure d'allophanoyle du XDI en excluant la quantité de solvant, de phosgène et d'HCl.

Selon une variante de réalisation selon laquelle le phosgène restant et le solvant ne sont pas extraits simultanément et dans laquelle le phosgène est extrait avant le solvant, l'étape b) selon l'invention peut s'effectuer pendant

l'extraction du phosgène ou après celle-ci, mais nécessairement avant la séparation d'avec le solvant.

Dans le cas de l'extraction simultanée du phosgène avec l'étape b), le procédé peut être présenté sous cette forme : procédé de préparation des xylylène diisocyanates XDI, en particulier du méta-xylylènediisocyanate mXDI, comprenant les étapes de

a) phosgénation de la xylylène-diamine XDA, en particulier de la m-xylylène diamine mXDA dans le cas du mXDI ;
b) extraction du phosgène et élimination simultanée de l'acide chlorhydrique du milieu réactionnel obtenu à l'étape a)

Dans le cas de l'extraction non simultanée du phosgène avec l'étape d'élimination, le procédé peut être présenté sous cette forme : procédé de préparation des xylylène diisocyanates XDI, en particulier du méta-xylylènediisocyanate mXDI, comprenant les étapes de

a) phosgénation de la xylylène-diamine XDA, en particulier de la m-xylylène diamine mXDA dans le cas du mXDI, suivie d'une étape d'extraction du phosgène ;
b) élimination de l'acide chlorhydrique du milieu réactionnel obtenu à l'étape a).

Dans le cas de procédés dont l'élimination du phosgène est antérieure ou simultanée à l'étape b), les étapes postérieures à l'étape b) sont des étapes d'élimination du solvant et/ou d'élimination des composés légers restants et/ou d'élimination du MXDI et/ou des composés lourds restants, ces étapes pouvant être ou non simultanées et réalisées dans n'importe quel ordre.

Dans tous les procédés selon l'invention, il est nécessaire d'avoir l'étape b) réalisée avant d'éliminer le solvant.

La teneur en chlorure d'allophanoyle de XDI est suivie par prélèvement d'un échantillon en bas de colonne 12 qui est dosé par chromatographie par perméation sur gel couplée à l'analyse de la diffusion d'un rayonnement infrarouge émis par exemple par un laser de puissance appropriée.

L'invention n'est pas limitée aux exemples de réalisation décrits et représentés. Par exemple, d'autres solvants peuvent être utilisés pour diluer le mélange réactionnel avant l'étape b); d'autres composés inertes peuvent être mis en oeuvre pour réaliser un stripping à contre-courant, croisé ou co-courant; des traversées en cascade à travers des suites de dispositifs membranaires ou à substrat solide peuvent être élaborées pour purifier le mélange réactionnel de l'acide chlorhydrique et transformer les espèces chimiques intermédiaires de préférence en XDI; d'autres conditions de dilution; d'autres méthodes de dosage de chlorure d'allophanoyle de XDI peuvent être utilisées (dosage potentiométrique, dosage par l'acide perchlorique, etc.).

**Exemples de réalisation**

Dans les exemples ci-dessous, la composition des différents mélanges a été mesurée par dosage après séparation du mélange sur une colonne séparative de type chromatographie (PL GEL 50Â 60CM 7,5 MM 5 μ suivie de PL GEL 50Â 60CM 7,5 MM 5 μ) de filtration sur gel dans un solvant tel que le dichlorométhane. La méthode de détection est l'infrarouge en mesurant la bande NCO à 2250 cm-1, après étalonnage avec le mXDI de concentration connue. Les résultats de composition sont donnés en poids en excluant la quantité de solvant, de phosgène et d'HCl.

Dans certains exemples, on peut doser les chlorures libérables (composés ayant au moins une fonction chlorure d'allophanoyle et/ou fonction chlorure de carbamyle) par une méthode dite de « Mesure de chlorure à froid ». Cette méthode décrit le dosage de chlorures libérables à une température comprise entre 20 et 25°C par titrage argentimétrique dans un milieu contenant de l'acide nitrique, de l'acétone du méthanol. Dans un bécher de 100 mL, peser une prise d'essai contenant selon la teneur un chlorure attendue :

$$\text{Prise d'essais (g)} = 0{,}071 / (\%\ \text{Cl attendu})$$

Ajouter 20 mL de méthanol, attendre 1 minute sous agitation, puis ajouter 3mL d'acide nitrique et 40mL d'acétone, puis titrer par de l'AgNO3 à 0,02N.

Soit V :Volume en mL de titrant coulé pour l'essai
T : Titre en mole/L de la solution de nitrate d'argent
E : Masse en g de l'échantillon
M : Masse molaire du Chlore
Résultat de la mesure de chlorure à froid :

$$Cl\ (mg/Kg) = (V \times T \times M \times 1000)/\ E$$

Les abréviations suivantes ont été utilisées :

ClBi : chlorométhyl-benzyl-isocyanate
mXDI : meta xylylene diisocyanate
QSP : Quantité suffisante pour
ARC : Accelerated Rate Calorimeter

Exemple 1 :

Dans un réacteur en verre de 1 L sont chargés : 351.9 g d'orthodichlorobenzène et 87.4 g de brut de mXDI issu de la phosgénation de la mXDA puis de sa déphosgénation. Le mélange est agité à 300 tour/min (agitation qui consiste en 4 pales inclinées et les contre pales) et est chauffé à 150 °C sous pression atmosphérique pendant 5 heures sous stripping d'argon.
Le brut de mXDI avant l'étape b) a la composition massique suivante : 1,7 % ClBi, 65 % mXDI, 2 % de dimère de mXDI, 6,7 % de chlorure d'allophanoyle de mXDI, 24,6 % de lourds à base de XDI (QSP 100).
Le produit final après l'étape b) a la composition massique suivante : 2,6 % ClBi, 74,5 % mXDI, 1,5 % de dimère de mXDI, 0 % de chlorure d'allophanoyle de mXDI, 21,4 % de lourds (QSP 100).

Pourcentage d'augmentation du mXDI suite à l'étape b) : ((% mXDI) après l'étape b)t - (% mXDI) avant l'étape b))/ (% mXDI) avant l'étape b) = +14,6 %

Exemple 2 :

Dans un réacteur en verre de 1 L sont chargés : 328,8 g de orthodichlorobenzène et 82,2 g de brut de mXDI issu de la phosgénation de la mXDA puis de sa déphosgénation. Le mélange est ensuite agité à 300 tour/min (agitation qui consiste en 4 pales inclinées et les contre pales) et est chauffé à 150 °C sous pression atmosphérique pendant 5,5 heures sous stripping d'argon.
Le brut de mXDI avant l'étape b) a la composition massique suivante : 1, % ClBi, 68,2 % mXDI, 1,5 % de dimère de mXDI, 5,9 % de chlorure d'allophanoyle de mXDI, 23 % de lourds (QSP 100).
Le produit final après l'étape b) a la composition massique suivante : 2,2 % ClBi, 77,1 % mXDI, 1,6 % de dimère de mXDI, 1,3 % de chlorure d'allophanoyle de mXDI, 17,8 % de lourds (QSP 100).

Pourcentage d'augmentation du mXDI suite à l'étape b) : ((% mXDI)après l'étape b) - (% mXDI)avant l'étape b) )/ (% mXDI)avant l'étape b) = +13,0 %

Exemple 3

Dans un réacteur en verre de 1 L sont chargés : 603,4 g de monochlorobenzène et 216,2 g de brut de mXDI issu de la phosgénation de la mXDA puis de sa déphosgénation. Le mélange est ensuite agité à 300 tour/min (agitation qui consiste en 4 pales inclinées et les contre pales) et est chauffé à 130 °C sous pression atmosphérique pendant 5,5 heures sous stripping d'argon.
Le brut de mXDI avant l'étape b) a la composition massique suivante : 1,6 % ClBi, 75,7 % mXDI, 4 % de dimère de mXDI, 4,7 % de chlorure d'allophanoyle de mXDI, 14 % de lourds (QSP 100).
Le produit final après l'étape b) a la composition massique suivante : 1,7 % ClBi, 78,4 % mXDI, 5 % de dimère de mXDI, 1,7 % de chlorure d'allophanoyle de mXDI, 13,2 % de lourds (QSP 100).
Pourcentage d'augmentation du mXDI suite à l'étape b) :

$$((\% \text{ mXDI})\text{après l'étape b)} - (\% \text{ mXDI})\text{avant l'étape b)})/(\% \text{ mXDI})\text{avant l'étape b)} = +3{,}6\ \%$$

Exemple 4 :

Dans un réacteur en verre de 1 L sont chargés : 612,3 g de monochlorobenzène et 202,2 g de brut de mXDI issu de la phosgénation de la mXDA puis de sa déphosgénation. Le mélange est ensuite agité à 300 tour/min (agitation qui consiste en 4 pales inclinées et les contre pales) et est chauffé à 130 °C sous pression atmosphérique pendant 11 heures sous stripping d'argon.

Le brut de mXDI avant l'étape b) a la composition massique suivante : 1,6 % ClBi, 75,7 % mXDI, 4 % de dimère de mXDI, 4,7 % de chlorure d'allophanoyle de mXDI, 14 % de lourds (QSP 100).

Le produit final après l'étape b) a la composition massique suivante : 1,7 % ClBi, 78,7 % mXDI, 3,8 % de dimère de mXDI, 0.9 % de chlorure d'allophanoyle de mXDI, 14,9 % de lourds (QSP 100).

Pourcentage d'augmentation du mXDI suite à l'étape b) :

$$((\% \text{ mXDI})\text{après l'étape b)} - (\% \text{ mXDI})\text{avant l'étape b)})/(\% \text{ mXDI})\text{avant l'étape b)} = +4{,}0\ \%$$

Mesure de chlorure à froid : avant l'étape b) 0,388 %m/m, après l'étape b) : 0,058%m/m.

Exemple 5 (stabilité thermique)

Exemple 5a. Un échantillon de mXDI brut, ayant subi l'étape b) conformément à l'invention, de composition massique (6,6 % d'orthodichlorobenzène, 2,4 % de ClBi, 69,6 % de mXDI, 1,4 % de dimère de mXDI, 0 % de chlorure d'allophanoyle de mXDI et 20 % de lourds) a été analysé par ARC. 4,19 g de mXDI brut ont été placés dans une cellule de 19,7265 g et l'essai a été réalisé selon une procédure heat-wait-search entre 50 °C et 425 °C avec des paliers tous les 3 °C. La température de début d'exothermie pour l'échantillon est de 185 °C.

Exemple 5b. Un échantillon de mXDI brut de composition massique (12,3 % d'orthodichlorobenzène, 2,2 % de ClBi, 68,7 % de mXDI, 2,6 % de dimère de mXDI, 3,1 % de chlorure d'allophanoyle de mXDI et 11 % de lourds) a été analysé par ARC. 3,841 g de mXDI brut ont été placés dans une cellule de 15,2940 g et l'essai a été réalisé selon une procédure heat-wait-search entre 50 °C et 425 °C avec des paliers tous les 3 °C. La température de début d'exothermie pour l'échantillon est de 167 °C.

La comparaison des exemples 5a et 5b montre que l'étape b) a un impact significatif sur la température de début de décomposition du produit. En effet, la température de début d'exothermie est 18 °C plus bas pour l'échantillon qui n'a pas été traité.

**Revendications**

**1.** Procédé de préparation des xylylène diisocyanates XDI, en particulier du méta-xylylènediisocyanate mXDI, comprenant les étapes de :

a. phosgénation de la xylylène-diamine XDA, en particulier de la m-xylylène diamine mXDA dans le cas du mXDI ;
b. élimination de l'acide chlorhydrique du milieu réactionnel obtenu à l'étape a) à une température comprise entre 120 et 190°C et à une pression comprise entre 1 mbar et 20 bars,
c. purification du XDI obtenu comprenant par exemple une étape d'élimination du $COCl_2$, une étape d'élimination du solvant de phosgénation, une étape d'élimination des composés lourds formés lors de la phosgénation et une étape d'élimination des espèces légères formées lors de la phosgénation,

dans lequel l'étape b) est maintenue jusqu'à ce que le dosage du milieu réactionnel résultant indique une teneur en chlorure d'allophanoyle de XDI, de préférence mXDI, inférieure à 3%, de préférence inférieure à 2%, avantageusement inférieure à 1%, en masse dans le milieu à réactionnel, en excluant la quantité de solvant, de phosgène et d'HCl.

**2.** Procédé selon la revendication 1, dans lequel au moins un solvant est ajouté au milieu réactionnel avant l'étape b), ledit solvant étant choisi parmi les solvants non réactifs avec les fonctions isocyanates, de préférence, le solvant utilisé pour l'étape de phosgénation, les alcanes, les chloroalcanes, les esters, les éthers, les aromatiques et les aromatiques halogénés.

**3.** Procédé selon la revendication 2, dans lequel le solvant ajouté au milieu réactionnel est en quantité supérieure à 30% en poids et avantageusement supérieure à 50% en poids.

**4.** Procédé selon l'une quelconque des revendications 1 à 3 dans lequel l'étape b) est mise en oeuvre dans une colonne de distillation.

**5.** Procédé selon l'une quelconque des revendications 1 à 3, dans lequel l'étape b) est mise en oeuvre par mise en contact à co-courant, à courant croisé ou à contre-courant du mélange réactionnel issu de la réaction de phosgénation et d'un composé inerte choisi parmi un composé de diazote, d'argon, de gaz carbonique et d'alcane léger de C1 à C4, à une température comprise entre 120°C et 190°C, de préférence entre 150°C et 190°C, et à une pression absolue comprise entre 1 et 5 bars, de préférence entre 1 et 3 bar.

**6.** Procédé selon l'une quelconque des revendications 1 à 4, dans lequel l'étape b) est réalisée par distillation dans une colonne (1) associée à un bouilleur (2) et un condenseur (3) avec, en tête de retrait (12), l'acide chlorhydrique résiduel et sensiblement pur, puis le retrait en pied de colonne (11) du mélange purifié.

**7.** Procédé selon l'une quelconque des revendications 1 à 3, dans lequel l'étape b) est réalisée par évaporation, en batch ou en continu, à une température comprise entre 120 et 190°C, de préférence entre 120°C et 140°C, de préférence entre 120 et 140°C et une pression comprise entre 10 et 1100 mbars absolus.

**8.** Procédé selon l'une quelconque des revendications 1 à 3, dans lequel l'étape b) est réalisé par piégeage chimique de l'acide chlorhydrique par des amines tertiaires immobilisées sur de la silice ou une base sans hydrogène mobile.

**9.** Procédé selon l'une quelconque des revendications 1 à 3, dans lequel l'étape d'élimination de l'acide chlorhydrique est mise en oeuvre par mise en contact du mélange réactionnel issu de la phosgénation avec un substrat solide (300) constitué par des zéolithes (310) ou des résines échangeuses d'ions, à une température comprise entre 120 et 190°C, de préférence entre 120 °C et 140 °C.

**10.** Procédé selon l'une quelconque des revendications 1 à 3, dans lequel l'étape d'élimination de l'acide chlorhydrique est réalisée par séparation membranaire (200).

**11.** Procédé selon la revendication 10, dans lequel la séparation est réalisée par pervaporation par vaporisation de l'acide chlorhydrique au travers d'une membrane organique ou céramique (210), à une température comprise entre 120 et 190°C, de préférence entre 120 et 160°C, et une pression partielle d'acide chlorhydrique dans le perméat comprise entre 0.1 et 100 mbar absolu.

**12.** Procédé selon la revendication 10, dans lequel une migration par perméation de l'acide chlorhydrique et une rétention des autres espèces sont réalisées au travers d'une membrane organique ou céramique, à une température comprise entre 120 et 190°C.

**13.** Procédé selon l'une quelconque des revendications 5 à 12, dans l'étape b) a lieu en batch ou en continu.

**14.** Composition de xylylène diisocyanate XDI, en particulier de méta-xylylènediisocyanate mXDI, **caractérisé en ce qu'**il est préparé selon l'une quelconque des revendications précédentes, et possède une teneur en chlorure d'allophanoyle inférieure à 3 %, de préférence inférieure à 2 %, et avantageusement inférieure à 1 %, et dans laquelle des résidus de solvant, de composés lourds et d'espèces légères sont encore présents en combinaison ou en mélange après séparation selon l'une quelconque des revendications 4 à 13 avec des teneurs inférieures à 5%, de préférence inférieures à 3%, et avantageusement inférieures à 1,5%.

## Patentansprüche

**1.** Verfahren zur Herstellung von Xylylen-Diisocyanaten XDI, insbesondere meta-Xylylen-Diisocyanat mXDI, das die

Schritte umfasst von:

a. Phosgenierung von Xylylendiamin XDA, insbesondere m-Xylylendiamin mXDA im Fall von mXDI;
a. Entfernen der Salzsäure aus dem in Schritt a) erhaltenen Reaktionsmedium bei einer Temperatur zwischen 120 und 190 °C und einem Druck zwischen 1 mbar und 20 bar,
a. Reinigen des erhaltenen XDI, die beispielsweise einen Schritt des Entfernens des $COCl_2$, einen Schritt des Entfernens des Phosgenierungslösungsmittels, einen Schritt des Entfernens der bei der Phosgenierung gebildeten schweren Verbindungen und einen Schritt des Entfernens der bei der Phosgenierung gebildeten leichten Spezies umfasst,

wobei Schritt b) aufrechterhalten wird, bis die Analyse des resultierenden Reaktionsmediums einen Allophanoylchloridgehalt an XDI, vorzugsweise mXDI, von weniger als 3 Gew.-%, vorzugsweise weniger als 2 Gew.-%, vorteilhafterweise weniger als 1 Gew.-% im Reaktionsmedium angibt, ohne die Menge an Lösungsmittel, Phosgen und HCl.

**2.** Verfahren nach Anspruch 1, wobei dem Reaktionsmedium vor Schritt b) mindestens ein Lösungsmittel zugesetzt wird, wobei das Lösungsmittel aus Lösungsmitteln ausgewählt ist, die nicht mit den Isocyanatfunktionen reagieren, vorzugsweise das für den Phosgenierungsschritt verwendete Lösungsmittel, Alkane, Chloralkane, Ester, Ether, Aromate und halogenierte Aromate.

**3.** Verfahren nach Anspruch 2, wobei das dem Reaktionsmedium zugesetzte Lösungsmittel in einer Menge von mehr als 30 Gew.-% und vorteilhafterweise mehr als 50 Gew.-% vorliegt.

**4.** Verfahren nach einem der Ansprüche 1 bis 3, wobei Schritt b) in einer Destillationskolonne durchgeführt wird.

**5.** Verfahren nach einem der Ansprüche 1 bis 3, wobei Schritt b) durchgeführt wird, indem das Reaktionsgemisch aus der Phosgenierungsreaktion im Gleichstrom, Kreuzstrom oder Gegenstrom mit einer inerten Verbindung in Kontakt gebracht wird, die ausgewählt ist aus einer Verbindung aus Distickstoff, Argon, Kohlendioxid und leichten C1- bis C4-Alkanen, bei einer Temperatur zwischen 120 °C und 190 °C, vorzugsweise zwischen 150 °C und 190 °C, und einem absoluten Druck zwischen 1 und 5 bar, vorzugsweise zwischen 1 und 3 bar.

**6.** Verfahren nach einem der Ansprüche 1 bis 4, wobei Schritt b) durch Destillation in einer Kolonne (1) durchgeführt wird, die mit einem Kessel (2) und einem Kondensator (3) verbunden ist, mit, am Entnahmekopf (12) restliche und im Wesentlichen reine Salzsäure, dann Entnahme des gereinigten Gemischs am Fuß der Kolonne (11).

**7.** Verfahren nach einem der Ansprüche 1 bis 3, wobei Schritt b) durch Verdampfen in Chargen oder kontinuierlich bei einer Temperatur zwischen 120 und 190 °C, vorzugsweise zwischen 120 °C und 140 °C, vorzugsweise zwischen 120 und 140 °C und einem Druck zwischen 10 und 1100 mbar absolut durchgeführt wird.

**8.** Verfahren nach einem der Ansprüche 1 bis 3, wobei Schritt b) durch chemisches Einfangen von Salzsäure durch tertiäre Amine, die auf Siliciumdioxid oder einer Base ohne mobilen Wasserstoff immobilisiert sind, durchgeführt wird.

**9.** Verfahren nach einem der Ansprüche 1 bis 3, wobei der Schritt des Entfernens der Salzsäure durch Inkontaktbringen des aus der Phosgenierung resultierenden Reaktionsgemischs mit einem festen Substrat (300), bestehend aus Zeolithen (310) oder ionenaustauscherharzen, bei einer Temperatur zwischen 120 und 190 °C, vorzugsweise zwischen 120 °C und 140 °C durchgeführt wird.

**10.** Verfahren nach einem der Ansprüche 1 bis 3, wobei der Schritt des Entfernens der Salzsäure durch Membrantrennung (200) durchgeführt wird.

**11.** Verfahren nach Anspruch 10, wobei die Trennung durch Pervaporation durch Verdampfung der Salzsäure durch eine organische oder keramische Membran (210) bei einer Temperatur zwischen 120 und 190 °C, vorzugsweise zwischen 120 und 160 °C, und einem Salzsäurepartialdruck im Permeat zwischen 0,1 und 100 mbar absolut durchgeführt wird.

**12.** Verfahren nach Anspruch 10, wobei eine Migration durch Permeation der Salzsäure und eine Rückhaltung der anderen Spezies durch eine organische oder keramische Membran bei einer Temperatur zwischen 120 und 190 °C durchgeführt werden.

**13.** Verfahren nach einem der Ansprüche 5 bis 12, wobei Schritt b) in Chargen oder kontinuierlich stattfindet.

**14.** Xylylen-Diisocyanat-XDI-Zusammensetzung, insbesondere meta-Xylylen-Diisocyanat mXDI, **dadurch gekennzeichnet, dass** sie nach einem der vorhergehenden Ansprüche hergestellt ist und einen Allophanoylchloridgehalt von weniger als 3 %, vorzugsweise weniger als 2 % und vorteilhafterweise weniger als 1 % aufweist, und wobei nach der Trennung nach einem der Ansprüche 4 bis 13 noch Reste an Lösungsmittel, an schweren Verbindungen und an leichten Spezies in Kombination oder im Gemisch vorhanden sind, mit Gehalten von weniger als 5 %, vorzugsweise weniger als 3 % und vorteilhafterweise weniger als 1,5 %.

## Claims

**1.** A method for producing xylylene diisocyanates XDI, in particular meta-xylylene diisocyanate mXDI, comprising the steps of:

a. phosgenation of xylylene diamine XDA, in particular m-xylylene diamine mXDA in the case of mXDI;
a. removal of the hydrochloric acid from the reaction medium obtained in step a) at a temperature of between 120 and 190°C and at a pressure of between 1 mbar and 20 bars,
a. purification of the XDI obtained comprising, for example, a step of removing the $COCl_2$, a step of removing the phosgenation solvent, a step of removing the heavy compounds formed during the phosgenation and a step of removing the light species formed during phosgenation,

wherein step b) is continued until the assay of the resulting reaction medium indicates an allophanoyl chloride content of XDI, preferably mXDI, of less than 3%, preferably less than 2%, advantageously less than 1%, by weight in the reaction medium, excluding the amount of solvent, phosgene and HCl.

**2.** The method according to claim 1, wherein at least one solvent is added to the reaction medium before step b), said solvent being chosen from solvents that are not reactive with the isocyanate functions, preferably the solvent used for the phosgenation step, alkanes, chloroalkanes, esters, ethers, aromatics and halogenated aromatics.

**3.** The method according to claim 2, wherein the solvent added to the reaction medium is in an amount greater than 30% by weight and advantageously greater than 50% by weight.

**4.** The method according to any one of claims 1 to 3, wherein step b) is implemented in a distillation column.

**5.** The method according to any one of claims 1 to 3, wherein step b) is implemented by bringing the reaction mixture resulting from the phosgenation reaction and an inert compound chosen from a compound of dinitrogen, argon, carbon dioxide and a light C1 to C4 alkane, into co-current, cross-current or counter-current contact, at a temperature between 120°C and 190°C, preferably between 150°C and 190°C, and at an absolute pressure of between 1 and 5 bar, preferably between 1 and 3 bar.

**6.** The method according to any one of claims 1 to 4, wherein step b) is carried out by distillation in a column (1) associated with a boiler (2) and a condenser (3) having, at the draw-off head (12), residual and substantially pure hydrochloric acid, then draw-off of the purified mixture at the bottom of the column (11).

**7.** The method according to any one of claims 1 to 3, wherein step b) is carried out by evaporation, in batches or continuously, at a temperature of between 120 and 190°C, preferably between 120°C and 140°C, preferably between 120 and 140°C and a pressure of between 10 and 1100 mbar absolute.

**8.** The method according to any one of claims 1 to 3, wherein step b) is carried out by chemical trapping of hydrochloric acid by tertiary amines immobilised on silica or a base without mobile hydrogen.

**9.** The method according to any one of claims 1 to 3, wherein the step of removing the hydrochloric acid is carried out by bringing the reaction mixture resulting from the phosgenation into contact with a solid substrate (300) consisting of zeolites (310) or ion exchange resins, at a temperature of between 120 and 190°C, preferably between 120°C and 140°C.

**10.** The method according to any of claims 1 to 3, wherein the step of removing hydrochloric acid is carried out by

membrane separation (200).

**11.** The method according to claim 10, wherein the separation is carried out by pervaporation by vaporisation of the hydrochloric acid through an organic or ceramic membrane (210), at a temperature of between 120 and 190°C, preferably between 120 and 160°C, and a partial pressure of hydrochloric acid in the permeate of between 0.1 and 100 mbar absolute.

**12.** The method according to claim 10, wherein migration by permeation of the hydrochloric acid and retention of the other species are carried out through an organic or ceramic membrane, at a temperature between 120 and 190°C.

**13.** The method according to any one of claims 5 to 12, wherein step b) takes place in batches or continuously.

**14.** A composition of xylylene diisocyanate XDI, in particular meta-xylylene diisocyanate mXDI, **characterised in that** it is prepared according to any one of the preceding claims, and has an allophanoyl chloride content of less than 3%, preferably less than 2%, and advantageously less than 1%, and wherein residues of solvent, of heavy compounds and of light species are still present in combination or in a mixture after separation according to any one of claims 4 to 13, with contents of less than 5%, preferably less than 3%, and advantageously less than 1.5%.

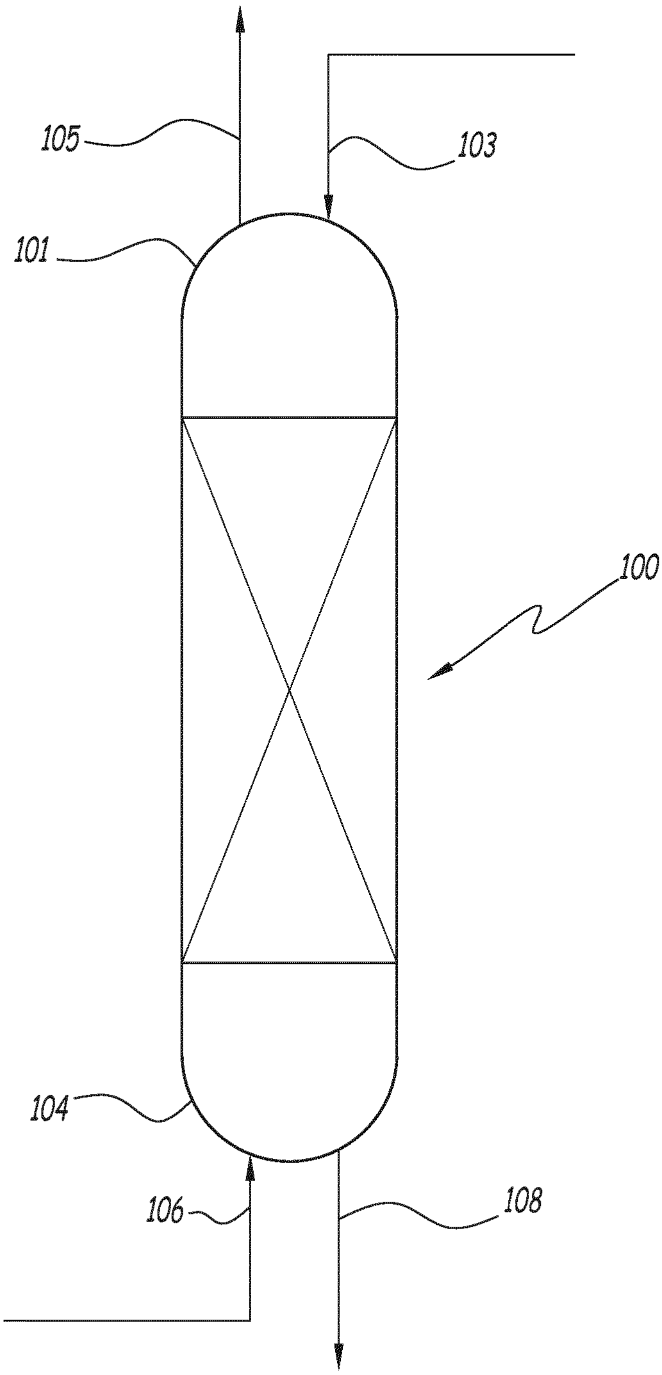


Fig.1

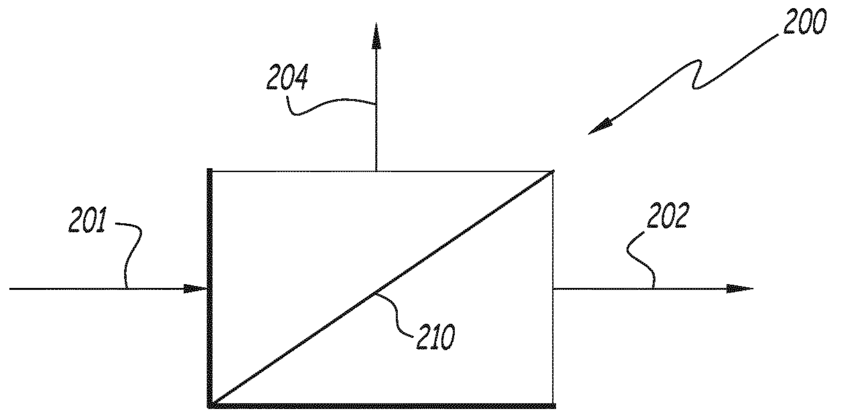


Fig.2

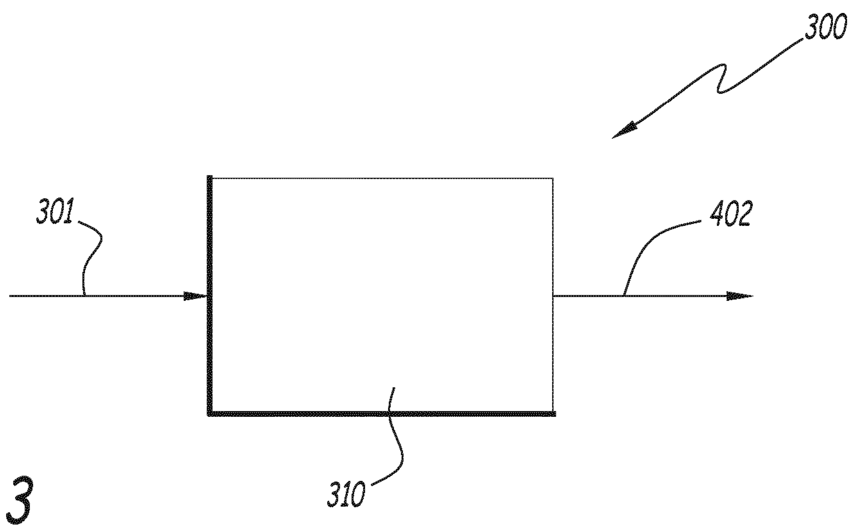


Fig.3

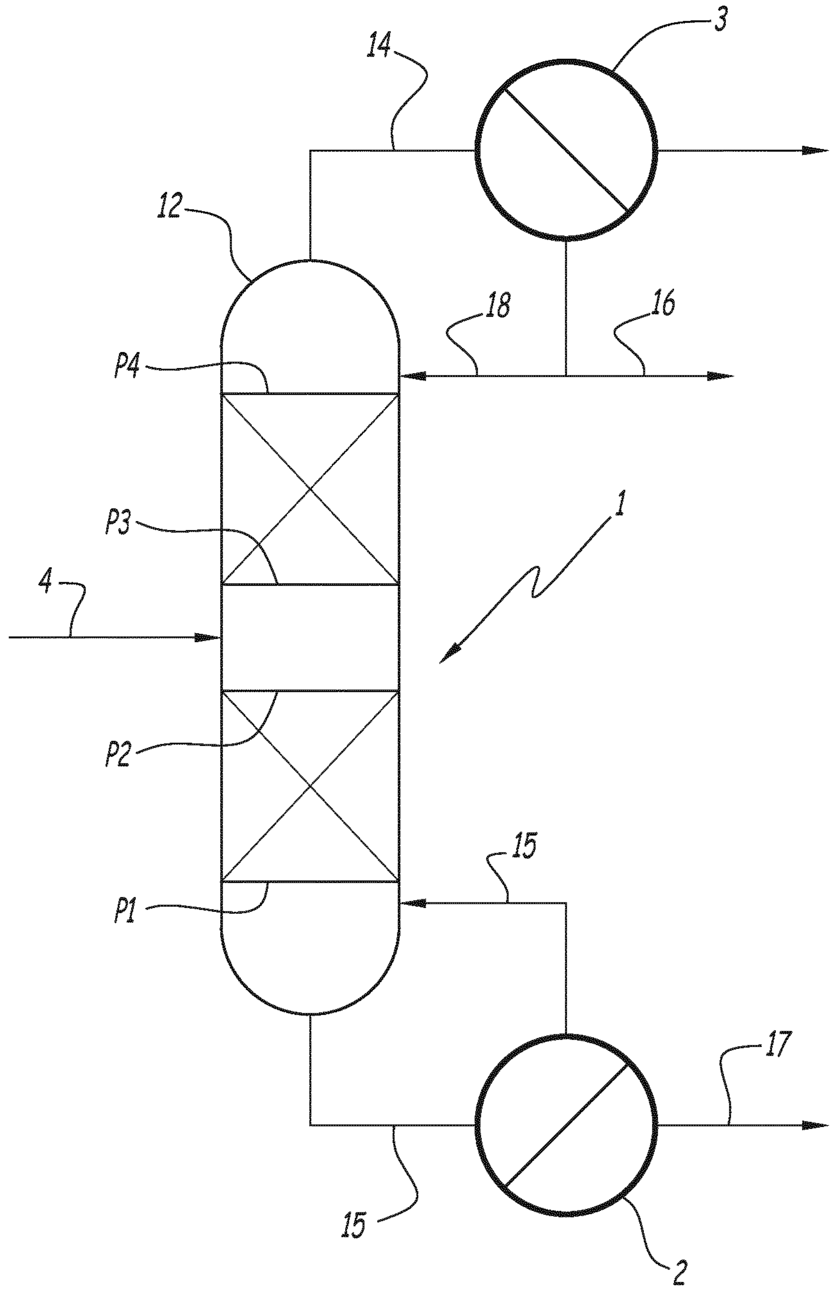


Fig.4

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- US 5523467 A
- CN 102070491
- GB 1350374 A
- US 3219678 A
- EP 0384463 A
- US 3470227 A
- FR 1555517
- GB 1119459 A
- FR 1555515